(19) Europäisches Patentamt
European Patent Office
Office européen des brevets

(11) **EP 4 188 318 B1**

(12) **EUROPEAN PATENT SPECIFICATION**

(45) Date of publication and mention
of the grant of the patent:
**06.03.2024   Bulletin 2024/10**

(21) Application number: **21743177.4**

(22) Date of filing: **14.07.2021**

(51) International Patent Classification (IPC):
*A61K 8/365* (2006.01)      *A61K 8/9783* (2017.01)
*A61K 8/49* (2006.01)       *A61K 36/185* (2006.01)
*A61Q 19/02* (2006.01)

(52) Cooperative Patent Classification (CPC):
**A61K 8/365; A61K 8/4973; A61K 8/9783;
A61K 36/284; A61Q 19/02**

(86) International application number:
**PCT/EP2021/069549**

(87) International publication number:
**WO 2022/023035 (03.02.2022 Gazette 2022/05)**

(54) **A PERSONAL CARE COMPOSITION COMPRISING ATRACTYLENOLIDE-I OR A SOURCE THEREOF**

KÖRPERPFLEGEZUSAMMENSETZUNG ATRACTYLENOLIDE-I ODER EINE QUELLE DES ATRACTYLENOLIDE-I

COMPOSITION DE SOINS PERSONNELS COMPRENANT L'ATRACTYLENOLIDE-I OU UNE SOURCE D'ATRACTYLENOLIDE-I

(84) Designated Contracting States:
**AL AT BE BG CH CY CZ DE DK EE ES FI FR GB
GR HR HU IE IS IT LI LT LU LV MC MK MT NL NO
PL PT RO RS SE SI SK SM TR**

(30) Priority: **30.07.2020   PCT/CN2020/105837
15.09.2020   EP 20196143**

(43) Date of publication of application:
**07.06.2023   Bulletin 2023/23**

(73) Proprietors:
• **Unilever IP Holdings B.V.
3013 AL Rotterdam (NL)**
Designated Contracting States:
**AL AT BE BG CH CZ DK EE ES FI FR GR HR HU
IS LI LT LU LV MC MK NL NO PL PT RO SE SI SK
SM**
• **Unilever Global IP Limited
Wirral, Merseyside CH62 4ZD (GB)**
Designated Contracting States:
**CY DE GB IE IT MT RS TR**

(72) Inventors:
• **GU, Xuelan
Shanghai, 200335 (CN)**
• **MI, Tingyan
Shanghai, 200335 (CN)**
• **XIAO, Xue
Shanghai, 200335 (CN)**
• **ZHANG, Hong
Shanghai, 200335 (CN)**

(74) Representative: **James, Helen Sarah
Unilever Patent Group
Bronland 14
6708 WH Wageningen (NL)**

(56) References cited:
AU-B2- 2013 202 356      KR-A- 20160 020 253
KR-B1- 101 613 175       KR-B1- 102 111 085

## Description

### Field of the Invention

The present invention relates to a personal care composition. In particular, the present invention relates to a personal care composition for providing benefits against hyperpigmentation.

### Background and Prior Art

People desire to look good and wish to maintain their appearance. However, most of the people develops various imperfections due to internal/inherent and external factors. Internal factors include ageing, conditions of particular skin e.g. dryness, blemish etc. On the other hand, external factors include sun light, pollution etc. Both of these factors induce skin imperfections which is not desired. Hence personal care compositions for improving skin appearance are quite popular. In particular, personal care compositions that includes one or more active from nature is in demand as people believe that compounds from nature are better for their overall skin health.

Hyperpigmentation, which is primarily caused by sunlight, is one of the imperfections that affects the skin appearance in considerable extent. Sunlight comprises harmful UV rays which is one of the main components responsible for hyperpigmentation. One of the ways to address the problem of hyperpigmentation is through cosmetic composition that reduces melanin contents in the skin. At the same time, consumers are increasingly relying on the natural ingredients in the cosmetic composition for skin benefits. Therefore, cosmetic composition with botanical ingredients are one of the major subject matter of interests for the cosmetic industry.

CN1 10420153 (Chifeng University, 2019) describes a Chinese medicinal composition for whitening and freckle removal, comprising *Rehemannia glutinosa*, Denrdobium, Dogwood, Medler, Schicandra, Peach kernel, Lotus roots, husk, Atractylodes, Tangerine peel, white Poria and Tremella. It further describes a Chinese medicinal cream and preparation thereof. The composition claimed to inhibit melanin deposition.

CN110664676 (Jian Royal Beauty Health Co Ltd., 2019) describes a cleansing and beauty water for skin comprising *Atractylodes chinensis* extract, *Tribulus terrestris* extract, Poria extract, Licorice extract, glycerine, and polyethylene glycol. It further describes that the water uses Chinese medicine formula, which is non-toxic, non-allergenic and less irritating to skin. It also states that the beauty water works as antioxidant and increases collagen fibre, hence delay skin aging.

CN1 10559195 (Guangzhou Oushudan Biological Tech Co Ltd., 2019) describes a mask powder having whitening and anti-aging function. It further describes that the mask powder comprises *Ganoderma lucidum*, rhodiola, *Gingko biloba*, Safflower, *Centella asiatica*, Ginseng, Atractylodes, Amaranth, white Aconite, Licorice, Angelica blossom, Pearl powder, Pine pollen, and Spirulina powder. It describes that the mask powder composition is guided by traditional medicine and provides glowing skin.

WO 2014/068470 (SEDERMA, 2014) describes a cosmetic active ingredient comprising extracts of at least three plants: *Astragalus membranaceus*, *Bupleurum faclcatum* and *Atractylodes macrocephala*. It further describes that the active ingredient can be incorporated in a topical composition and can be used to improve the appearance by treating and preventing sign of aging and imperfection of the skin.

US 2009/0041875 (Shiseido Company Ltd., 2009) describes a novel anti-wrinkle composition comprising one or more from among Juniper, Baizhu and Coltsfoot extracts as active ingredients.

AU2013202356B describes skin lightening compositions comprising 12-hydroxystearic acid.

KR 20160020253 describes skin whitening compositions comprising atractylenolide-I.

Although prior art documents describe compositions comprising various botanical extracts, however, still there is a need to provide a personal care composition comprising active botanical ingredient for providing enhanced benefit against hyperpigmentation.

### Objects of the invention

In view of the foregoing, it is an object of the present invention to provide a skin care composition.

It is another object of the present invention to provide a skin care composition for improving appearance of skin.

It is yet another object of the present invention to provide a skin care composition for reducing hyperpigmentation.

The present inventors have surprisingly found that a personal care composition comprising Atractylenolide- I and a hydroxy fatty acid provides synergistic benefit against hyperpigmentation and improves skin appearance and thereby satisfying one or more of the above-mentioned objects.

## Summary of the invention

In a first aspect, the present invention provides a personal care composition comprising:

a. Atractylenolide-I or a source of Atractylenolide-I; and

b. A hydroxy fatty acid having the number of carbon atom from 12 to 20, wherein the amount of hydroxy fatty acid is in the range of 0.1 to 20% by weight of the composition, and the amount of Atractylenolide-I is in the range of 0.01 to 15% by weight of the composition.

In a second aspect, the present invention provides a personal care composition comprising:

a. An extract of *Atractylenolide* macrocephala comprising Atractylenolide-I; and

b. A hydroxy fatty acid having the number of carbon atom from 12 to 20, wherein the amount of hydroxy fatty acid is in the range of 0.1 to 20% by weight of the composition, and the amount of Atractylenolide-I is in the range of 0.01 to 15% by weight of the composition.

In a third aspect, the present invention provides a method for reducing hyperpigmentation on skin comprising the step of applying a composition according to the first aspect.
In a fourth aspect, the present invention provides the use of a composition according to the first aspect for reducing hyperpigmentation on skin.
In a fifth aspect, the present invention provides the use of a composition comprising Atractylenolide-I or a source of Atractylenolide-I and a hydroxy fatty acid having the number of carbon atom from 12 to 20 for reducing herimentation on skin, wherein the amount of hydroxy fatty acid is in the range of 0.1 to 20% by weight of the composition, and the amount of Atractylenolide-I is in the range of 0.01 to 15% by weight of the composition.
These and other aspects, features and advantages will become apparent to those of ordinary skill in the art from a reading of the following detailed description. For the avoidance of doubt, any feature of one aspect of the present invention may be utilized in any other aspect of the invention. The word "comprising" is intended to mean "including" but not necessarily "consisting of" or "composed of." In other words, the listed steps or options need not be exhaustive. It is noted that the examples given in the description below are intended to clarify the invention and are not intended to limit the invention to those examples *per se*. Similarly, all percentages are weight/weight percentages unless otherwise indicated. Except in the operating and comparative examples, or where otherwise explicitly indicated, all numbers in this description indicating amounts of material or conditions of reaction, physical properties of materials and/or use are to be understood as modified by the word "about". Numerical ranges expressed in the format "from x to y" are understood to include x and y. When for a specific feature multiple preferred ranges are described in the format "from x to y", it is understood that all ranges combining the different endpoints are also contemplated.

## Detailed description of the invention

The disclosure of the invention as found herein is to be considered to cover all embodiments as found in the claims as being multiply dependent upon each other irrespective of the fact that claims may be found without multiple dependency or redundancy.
Where a feature is disclosed with respect to a particular aspect of the invention (for example a composition of the invention), such disclosure is also to be considered to apply to any other aspect of the invention (for example a method of the invention) *mutatis mutandis.*
By "A Personal Care Composition" as used herein, is meant to include a composition for topical application to the skin of mammals, especially humans. Such a composition may be generally classified as leave-on or rinse off but is preferably of the leave on type. The composition is formulated into a product which is applied to a human body specifically for improving appearance but may also be capable of providing cleansing, odour control or general aesthetics. The composition of the present invention can be in the form of a liquid, lotion, cream, foam, scrub, gel, or toner, or applied with an implement or via a face mask or a pad. Non-limiting examples of such compositions include leave-on skin lotions, creams, antiperspirants, deodorants, lipsticks, foundations, mascara, sunless tanners and sunscreen lotions. The composition of the present invention is preferably a leave-on composition. "Skin" as used herein is meant to include skin on the face and body (e.g., neck, chest, back, arms, underarms, hands, legs, buttocks and scalp) and especially to the sun exposed parts thereof.
According to present invention there is provided a personal care composition for reducing hyperpigmentation. The composition comprises Atractylenolide-I or a source of Atractylenolide-I and a hydroxy fatty acid having the number of carbon atom from 12 to 20.

Atractylenolide-I is a botanical active, found in the rhizome of a plant of Atractylodes origin. The term "botanical active" preferably refers to a compound present in a plant which provides a particular benefit e.g. pain relief, anti-inflammation benefit, work as antioxidant etc. In practice, botanical actives are extracted from the plant or in some cases plant extract itself is used in a composition for providing benefits.

The term "source" used hereabove preferably refers to a substance comprising the active ingredient. In case of present invention, the sources of Atractylenolide-I include an extract derived from a plant or parts of a plant such as, roots, rhizome, stem, leaf, flower etc comprising Atractylenolide-1. It also includes any blend or mixture comprising Atractylenolide-I as one of the ingredients

The amount of Atractylenolide-I is in the range of 0.01 to 15% by weight, more preferably 0.02 to 10% by weight, even more preferably 0.05 to 7.5% by weight and most preferably 0.1 to 5% by weight of the composition. When a source of Atractylenolide-I is used in the composition, concentration of the source should be adjusted to aforesaid range depending on active level of Atractylenolide-I in the source.

Preferably the Atractylenolide-I in the composition is sourced from a plant extract. A preferred plant extract is *Atractylenolide macrocephala* or Sai-bai-tang (SBT) extract. Preferably the Atractylenolide-I is sourced from rhizome of *Atractylenolide macrocephala.*

The composition of the present invention further comprises a hydroxy fatty acid. Hydroxy fatty acids are derivatives of fatty acids containing a hydroxyl group at one or more positions. Preferably the fatty acids are saturated or unsaturated and branched or unbranched. The hydroxy fatty acids used in the present invention having 12 to 20 carbon atoms, preferably 14 to 20 carbon atoms, more preferably 16 to 20 carbon atoms, most preferably 18 to 20 carbon atoms. Preferably the hydroxy fatty acid is selected from 2-hydroxymyristic acid, 3-hydroxymyristic acid, 2-hydroxypalmitic acid, 3-hydroxypalmitic acid, 12-hydroxystearic acid, 17-hydorxystearic acid etc.

Preferably the hydroxy fatty acid is hydroxystearic acid. The most preferred hydroxystearic acid is 12 hydroxystearic acid (12HSA).

In case of present invention, the hydroxy fatty acid is combined with the Atractylenolide-I or source of Atractylenolide-I for providing synergistic benefit against hyperpigmentation on skin.

The amount of hydroxy fatty acid is in the range of 0.1 to 20% by weight, more preferably 0.2 to 15% by weight and furthermore preferably 0.5 to 10%, even more preferably 1 to 10%, yet more preferably 2 to 10% and most preferably 2 to 10% by weight of the composition.

Preferably, the molar ratio of the Atractylenolide-I to hydroxy fatty acid in the composition is in the range of 1:80 to 80:1, more preferably 1:30 to 30:1, even more preferably 1:10 to 10:1 and most preferably 1:3 to 3:1.

The composition preferably comprises one or more organic sunscreens. A wide variety of organic sunscreen is suitable for use in combination with the essential ingredients of this invention. Suitable UV-A / UV-B sunscreen include, 2-hydroxy-4-methoxybenzophenone, octyldimethyl p-aminobenzoic acid, digalloyltrioleate, 2,2-dihydroxy-4-methoxybenzophenone, ethyl-4-(bis(hydroxypropyl)) aminobenzoate, 2-ethylhexyl-2-cyano-3,3-diphenylacrylate, 2-ethylhexylsalicylate, glyceryl p-aminobenzoate, 3,3,5-trimethylcyclohexylsalicylate, methylanthranilate, p-dimethyl- aminobenzoic acid or aminobenzoate, 2-ethylhexyl-p-dimethyl- amino-benzoate, 2-phenylbenzimidazole-5-sulfonic acid, 2-(p-dimethylaminophenyl)-5-sulfonicbenzoxazoic acid, 2-ethylhexyl-p-methoxycinnamate, butylmethoxydibenzoylmethane, 2-hydroxy-4- methoxybenzophenone, octyldimethyl-p-aminobenzoic acid and mixtures thereof. The most suitable organic sunscreens are 2-ethylhexyl-p-methoxycinnamate, butylmethoxydibenzoylmethane or a mixture thereof.

A safe and effective amount of organic sunscreen may be used in the compositions useful in the subject invention. The composition preferably comprises from 0.1% to 15% by weight, more preferably from 0.1% to 10% by weight of organic sunscreen.

The composition of the invention preferably comprises an additional skin benefit agent. Preferably, the skin benefit agent is selected from niacinamide, nicotinic acid or niacinamide, isonicotinamide, picolinamide, hexyl resorcinol and mixtures thereof. The skin benefit agents, when used, are preferably present in an amount in the range of 0.1 to 15% by weight, more preferably 0.2 to 10% by weight, even more preferably 0.5 to 5% by weight of the composition.

The composition may comprise other beneficial skin care actives like retinol, retinyl esters, resorcinol, allantoin, ubiquinone, conjugated linoleic acid or derivatives thereof. Of these the most preferred ones for inclusion in the composition of the invention are anti-aging actives like retinol or retinyl esters.

Compositions of the present invention will also include a cosmetically acceptable carrier which is a water and oil emulsion, which in certain embodiments may be water-in-oil emulsion. Preferred emulsions, however, are the oil-in-water variety.

Preferred hydrophobic material for use in the oil phase of such emulsions includes emollients such as fats, oils, fatty alcohols, fatty acids, soaps, silicone oils, synthetic esters and/or hydrocarbons.

Silicones may be divided into the volatile and non-volatile variety. Volatile silicone oils (if used) are preferably chosen from cyclic (cyclomethicone) or linear polydimethylsiloxanes containing from 3 to 9, preferably from 4 to 5, silicon atoms.

Non-volatile silicones useful as an emollient material include polyalkyl siloxanes, polyalkylaryl siloxanes and polyether siloxane copolymers. The essentially non-volatile polyalkyl siloxanes useful herein include, for example, polydimethyl siloxanes with viscosities of from about $5 \times 10^{-6}$ to $0.1$ m$^2$/s at 25 °C. Among the preferred non-volatile emollients useful in the present compositions are the polydimethyl siloxanes having viscosities from about $1 \times 10^{-5}$ to about $4 \times 10^{-4}$ m$^2$/s at 25 °C.

Specific examples of non-silicone emollients include stearyl alcohol, glyceryl monoricinoleate, mink oil, cetyl alcohol, isopropyl isostearate, stearic acid, isobutyl palmitate, isocetyl stearate, oleyl alcohol, isopropyl laurate, hexyl laurate, decyl oleate, octadecan-2-ol, isocetyl alcohol, eicosanyl alcohol, behenyl alcohol, cetyl palmitate, silicone oils such as dimethylpolysiloxane, di-n-butyl sebacate, isopropyl myristate, isopropyl palmitate, isopropyl stearate, butyl stearate, polyethylene glycol, triethylene glycol, lanolin, cocoa butter, corn oil, cotton seed oil, olive oil, palm kernel oil, rape seed oil, safflower seed oil, evening primrose oil, soybean oil, sunflower seed oil, avocado oil, sesame seed oil, coconut oil, arachis oil, castor oil, acetylated lanolin alcohols, petroleum jelly, mineral oil, butyl myristate, isostearic acid, palmitic acid, isopropyl linoleate, lauryl lactate, myristyl lactate, decyl oleate, myristyl myristate, and mixtures thereof.

Among the ester emollients are:

a) Alkenyl or alkyl esters of fatty acids having 10 to 20 carbon atoms. Examples thereof include isoarachidyl neopentanoate, isodecyl neopentanoate, isononyl isonanoate, cetyl ricinoleate, oleyl myristate, oleyl stearate, and oleyl oleate;
b) Ether-esters such as fatty acid esters of ethoxylated fatty alcohols;
c) Polyhydric alcohol esters. Butylene glycol, ethylene glycol mono and di-fatty acid esters, diethylene glycol mono- and di-fatty acid esters, polyethylene glycol (200-6000) mono- and di-fatty acid esters, propylene glycol mono- and di-fatty acid esters, polypropylene glycol 2000 monooleate, polypropylene glycol 2000 monostearate, ethoxylated propylene glycol monostearate, glyceryl mono- and di-fatty acid esters, polyglycerol poly-fatty esters, ethoxylated glyceryl monostearate, 1,3-butylene glycol monostearate, 1,3-butylene glycol distearate, polyoxyethylene polyol fatty acid ester, sorbitan fatty acid esters, and polyoxyethylene sorbitan fatty acid esters are satisfactory polyhydric alcohol esters. Particularly useful are pentaerythritol, trimethylolpropane and neopentyl glycol esters of $C_1$-$C_{30}$ alcohols. An Example is pentaerythrityl tetraethylhexanoate;
d) Wax esters such as beeswax, spermaceti wax and tribehenin wax;
e) Sterols esters, of which cholesterol fatty acid esters are examples thereof;
f) Sugar ester of fatty acids such as sucrose polybehenate and sucrose polycottonseedate; or
g) mixtures of two or more of the foregoing (a) to (f).

Of particular use also are the $C_{12-15}$ alkyl benzoate esters sold under the Finsolv® brand.

Hydrocarbons which are suitable emollients include petrolatum, mineral oil, $C_{11}$-$C_{13}$ isoparaffins, polyalphaolefins, isohexadecane or a mixture thereof.

Amounts of water in the carrier may, for example, range from 1 to 99%, more preferably from 5 to 90%, even more preferably from 35 to 80%, optimally between 40 and 70% by weight of the personal care composition.

Other materials which can be included in the cosmetically acceptable carrier include solvents, humectants, thickeners and powders. Examples of each of these types of material, which can be used singly or as mixtures, are as follows: Solvents include ethyl alcohol, isopropanol, acetone, ethylene glycol monoethyl ether, diethylene glycol monobutyl ether, diethylene glycol monoethyl ether and mixtures thereof.

Humectants include those of the polyhydric alcohol-type. Typical polyhydric alcohols include polyalkylene glycols and more preferably alkylene polyols and their derivatives, including propylene glycol, dipropylene glycol, polypropylene glycol, polyethylene glycol and derivatives thereof, sorbitol, hydroxypropyl sorbitol, hexylene glycol, 1,3-butylene glycol, isoprene glycol, 1,2,6-hexanetriol, glycerol, ethoxylated glycerol, propoxylated glycerol and mixtures thereof. The amount of humectant may range, for example, anywhere from 0.5 to 50%, more preferably between 1 and 15% by weight of the composition. Most preferred is glycerol (also known as glycerin). Amounts of glycerin may range, for example, from 0.5% to 50%, more preferably from 1 to 35%, optimally from 2 to 15% by weight of the composition.

A variety of thickening agents may be included in the compositions. Illustrative but not limiting are stearic acid, Acrylamide/Sodium Acryloyldimethyltaurate Copolymer (Aristoflex® AVC), Hydroxyethyl Acrylate/Sodium Acryloyldimethyltaurate Copolymer, Aluminum Starch Octenyl Succinate, Polyacrylates (such as Carbomers including Carbopol® 980, Carbopol® 1342, Pemulen TR-2® and the Ultrez® thickeners), Polysaccharides (including xanthan gum, guar gum, pectin, carageenan and sclerotium gums), celluloses (including carboxymethyl cellulose, ethyl cellulose, hydroxyethyl cellulose and methyl hydroxymethyl cellulose), minerals (including talc, silica, alumina, mica and clays, the latter being represented by bentonites, hectorites and attapulgites), magnesium aluminum silicate and mixtures thereof. Amounts of the thickeners may range, for example, from 0.05 to 10%, more preferably from 0.3 to 2% by weight of the composition.

Powders include chalk, talc, Fullers earth, kaolin, starch, gums, colloidal silica sodium polyacrylate, tetraalkyl and/or trialkyl aryl ammonium smectites, chemically modified magnesium aluminium silicate, organically modified montmorillonite clay, hydrated aluminium silicate, fumed silica, carboxyvinyl polymer, sodium carboxymethyl cellulose and ethylene glycol monostearate.

The personal care composition of this invention is preferably a skin care composition. More preferably, the composition is preferably an antiperspirant composition or a face (except eye lids and lips) care composition. The skin care composition refers to a composition suitable for topical application to human skin, including leave-on and wash-off products. Preferably the term encompasses a fluid or liquid, and particularly a moisturizer rather than a make-up product. Most preferred are leave-on compositions. The term "leave-on" as used with reference to compositions herein means a composition that is applied to or rubbed on the skin and left thereon. The term "wash-off" as used with reference to compositions herein means a skin cleanser that is applied to or rubbed on the skin and rinsed off substantially immediately subsequent to application. The term "skin" as used herein includes the skin on the face (except eye lids and lips), neck, chest, abdomen, back, arms, under arms, hands, and legs. Preferably the term "skin" includes the skin on the face (except eye lids and lips) and under arms. More preferably it means skin on the face other than lips and eyelids.

The composition can be formulated in any known format, more preferred formats being creams, lotions or gel.

Packaging for the composition of this invention can be a jar or tube as well as any other formats typically seen for cosmetic, cream, washing and lotion type products. The compositions may be applied topically and preferably 1-4 milligrams of composition is applied per square centimeter of skin.

The composition of the invention preferably delivers a cosmetic benefit to the skin of an individual to which it is topically applied. Examples of cosmetic benefits include reducing the appearance of fine lines, wrinkles, pores and/or blemish spots; evening skin tone, long lasting optical effect or a combination thereof on the desired skin surface.

According to present invention, there is provided a personal care composition comprising an extract of Atractylenolide macrocephala and a hydroxy fatty acid having the number of carbon atom from 12 to 20.

According to present invention, there is provided a method for improving the appearance of skin by reducing hyperpigmentation on skin. The method comprises the step of applying a composition according to the first aspect on the desired skin surface.

According to present invention, there is provided a use of a composition according to the first aspect for reducing hyperpigmentation on skin.

According to present invention, there is provided a use of a composition comprising Atractylenolide-I or a source of Atractylenolide-I and a hydroxy fatty acid having number of carbon atom from 12 to 20 for reducing hyperpigmentation on skin.

The present invention will now be demonstrated by way of non-limiting examples below. The examples are for illustration only and do not limit the scope of the invention in any manner.

**Examples:**

Materials:

| Abbreviations | Full form | Supplier |
|---|---|---|
| AT-I | Atractylenolide -1 | Tauto (Cat No. E-0114) |
| 12 HSA | 12 hydroxystearic acid | Sigma (Cat No.219967-1G) |

Process of preparing an extract of *Atractylodes macrocephala*:

An aqueous co-extract of the Atractylodes macrocephala (root, sourced from China) was prepared by the usual water-reflux process in which the duration of each reflux cycle was 30 minutes. There were two such cycles. A rotary evaporator maintained at 60°C was used for vacuum distillation. The aqueous extract was freeze-dried into a powder.

Method for cell culture and treatment:

Normal human epidermal melanocytes (NHEM) (Biocell, Xi'an, China) were grown in melanocyte growth medium (MGM, Promocell, Germany) with human melanocyte growth supplement (Promocell, Germany). NHEM cells were seeded at a density of $4 \times 10^4$ cells in 500 μL MGM/well in a 24-well plate and incubated for 48 hours in an incubator (Thermo Scientific, Model 3111) at 37°C with 5% $CO_2$. After 48 hours, media was replaced with fresh media containing test samples (as per Table 1) for the following 72 hours.

Method for cytotoxicity assessment:

At the end of the 72 hours incubation period, cell viability was determined using the Cell Counting Kit-8 (CCK8) method (Dojindo, Japan). Briefly, cells were washed once with phosphate buffered saline (PBS) solution. Freshly prepared 10% CCK8 solution was added into each well including the blank control (vehicle control) wells without cells. Plates were incubated for 2 hrs at 37°C in the $CO_2$ incubator. Absorbance was then measured at 450 nm using TECAN Safire II plate reader.

It was found that all the concentration used in Table 1 are within the Cytotoxicity limit. This means none of the concentration used in the examples are cytotoxic.

Method for melanin content quantification:

After CCK8 absorbance readings were obtained, cells were lysed by lysis buffer at 60°C for 1 hr. Then, 50μL cell lysate was transferred to a new 96-well plate and absorbance was measured at 405 nm using TECAN plate reader. Serial dilutions of pure melanin were prepared as references.

The melanin content was calculated from the melanin standard curve. The melanin concentrations (μg/mL) were then normalized to the relative cell viability. The melanin inhibition rates were then calculated using the following equation:

$$\% \ Inhbition = \frac{Normalized \ melanin \ content \ (Vehicle \ control) - Normalized \ melanin \ content \ (Test \ sample)}{Normalized \ melanin \ content \ (vehicle \ control)} \times 100\%$$

Melanin inhibition data are summarized below in Table 2.

Table 2:

| Example | Treatment | Melanin inhibition rate (% inhibition) |
|---|---|---|
| A | Vehicle Control | 0±9.38 |
| B | 12HSA 5 μM | 5.6±3.03 |
| C | SBT 100 ppm | 16.9±2.26 |
| D | AT-I 5 μM | 7.8±2.80 |
| 1 | SBT 100 ppm +12HSA 5 μM | 38.2±6.21 |
| 2 | AT-I 5 μM +12HSA 5 μM | 31.0±9.11 |

From the above table it is evident that the examples within the scope of the present invention (Example 1 and 2) provide synergistic inhibition of melanin content in melanocyte when compared with the examples that are outside the scope of the present invention (Example B, C and D).

Therefore, from the above description it is evident that the present invention provides a personal care composition that inhibits melanin content and thereby reduce hyperpigmentation on the skin appearance of individuals.

**Claims**

1. A personal care composition comprising:

   a. Atractylenolide-I or a source of Atractylenolide-I; and
   b. a hydroxy fatty acid having the number of carbon atom from 12 to 20, wherein the amount of hydroxy fatty acid is in the range of 0.1 to 20% by weight of the composition, and the amount of Atractylenolide-I is in the range of 0.01 to 15% by weight of the composition.

2. The composition as claimed in claim 1 wherein the amount of Atractylenolide-I is in the range of 0.02 to 10% by weight, preferably 0.1 to 5% by weight of the composition.

3. The composition as claimed in claim 1 or 2 wherein the source of Atractylenolide-I is a plant extract.

4. The composition as claimed in claim 3 wherein the plant is *Atractylodes macrocephala.*

5. The composition as claimed in any one of the preceding claims wherein the amount of hydroxy fatty acid is in the range of 1 to 10% by weight of the composition.

6. The composition as claimed in any one of the preceding claims wherein the hydroxy fatty acid is hydroxystearic acid.

7. The composition as claimed in claim 6 wherein the hydroxystearic acid is 12-hydroxystearic acid (12HSA).

8. The composition as claimed in any one of the preceding claims wherein the composition further comprises an additional skin benefit agent.

9. The composition as claimed in claim 8 wherein the skin benefit agent is selected from niacinamide, picolinamide, isonicotinamide, hexyl resorcinol and mixture thereof.

10. The composition as claimed in any one of the preceding claims wherein the composition comprises from 0.1 to 15% by weight of an organic sunscreen.

11. The composition as claimed in any one of the preceding claims wherein the composition is a leave-on composition in the form of a cream, lotion or gel.

12. A personal care composition comprising;

    a. an extract of *Atractylodes* macrocephala comprising Atractylenolide-I; and
    b. a hydroxy fatty acid having the number of carbon atom from 12 to 20 wherein the amount of hydroxy fatty acid is in the range of 0.1 to 20% by weight of the composition, and the amount of Atractylenolide-I is in the range of 0.01 to 15% by weight of the composition.

13. A non-therapeutic method for reducing hyperpigmentation on skin comprising the step of applying a composition as claimed in any one of claims 1 to 12.

14. A non-therapeutic use of a composition as claimed in any one of claims 1 to 12 for reducing hyperpigmentation on skin.

15. Use of a composition comprising:

    a. Atractylenolide-I or a source of Atractylenolide-I; and
    b. a hydroxy fatty acid having the number of carbon atom from 12 to 20, for reducing hyperpigmentation on skin,

    wherein the amount of hydroxy fatty acid is in the range of 0.1 to 20% by weight of the composition, and the amount of Atractylenolide-I is in the range of 0.01 to 15% by weight of the composition.

**Patentansprüche**

1. Körperpflegezusammensetzung, umfassend:

    a. Atractylenolid-I oder eine Quelle von Atractylenolid-I; und
    b. eine Hydroxyfettsäure mit der Anzahl der Kohlenstoffatome von 12 bis 20, wobei die Menge an Hydroxyfettsäure in dem Bereich von 0,1 bis 20% Gewichts-% der Zusammensetzung und die Menge an Atractylenolid-I im Bereich von 0,01 bis 15 Gewichts-% der Zusammensetzung liegt.

2. Zusammensetzung, wie im Anspruch 1 beansprucht, wobei die Menge an Atractylenolid-I im Bereich von 0,02 bis 10 Gewichts-%, vorzugsweise von 0,1 bis 5 Gewichts-% der Zusammensetzung liegt.

3. Zusammensetzung, wie im Anspruch 1 oder 2 beansprucht, wobei die Quelle des Atractylenolid-I ein Pflanzenextrakt ist.

4. Zusammensetzung, wie im Anspruch 3 beansprucht, wobei die Pflanze Atractylodes macrocephala ist.

5. Zusammensetzung, wie in irgendeinem der vorhergehenden Ansprüche beansprucht, wobei die Menge an Hydroxyfettsäure im Bereich von 1 bis 10 Gewichts-% der Zusammensetzung liegt.

6. Zusammensetzung, wie in irgendeinem der vorhergehenden Ansprüche beansprucht, wobei die Hydroxyfettsäure Hydroxystearinsäure ist.

7. Zusammensetzung, wie im Anspruch 6 beansprucht, wobei die Hydroxystearinsäure 12-Hydroxystearinsäure (12HSA) ist.

8. Zusammensetzung, wie in irgendeinem der vorhergehenden Ansprüche beansprucht, wobei die Zusammensetzung außerdem einen zusätzlichen hautfördernden Wirkstoff umfasst.

9. Zusammensetzung, wie im Anspruch 8 beansprucht, wobei der hautfördernde Wirkstoff aus Niacinamid, Picolinamid, Isonicotinamid, Hexylresorcin und einer Mischung davon ausgewählt ist.

10. Zusammensetzung, wie in irgendeinem der vorhergehenden Ansprüche beansprucht, wobei die Zusammensetzung 0,1 bis 15 Gewichts-% eines organischen Sonnenschutzmittels umfasst.

11. Zusammensetzung, wie in irgendeinem der vorhergehenden Ansprüche beansprucht, wobei die Zusammensetzung eine Leave-On-Zusammensetzung in Form einer Creme, Lotion oder eines Gels ist.

12. Körperpflegezusammensetzung, umfassend:

a. einen Extrakt von Atractylodes macrocephala, umfassend Atractylenolid-I; und
b. eine Hydroxyfettsäure mit der Anzahl der Kohlenstoffatome von 12 bis 20, wobei die Menge an Hydroxyfettsäure im Bereich von 0,1 bis 20 Gewichts-% der Zusammensetzung und die Menge an Atractylenolid-I im Bereich von 0,01 bis 15 Gewichts-% der Zusammensetzung liegt.

13. Nicht-therapeutisches Verfahren zur Verminderung der Hyperpigmentierung auf der Haut, umfassend den Schritt der Anwendung einer Zusammensetzung, wie in irgendeinem der Ansprüche 1 bis 12 beansprucht.

14. Nicht-therapeutische Verwendung einer Zusammensetzung, wie in irgendeinem der Ansprüche 1 bis 12 beansprucht, zur Verminderung der Hyperpigmentierung auf der Haut.

15. Verwendung einer Zusammensetzung, umfassend:

a. Atractylenolid-I oder eine Quelle von Atractylenolid-I; und
b. eine Hydroxyfettsäure mit der Anzahl der Kohlenstoffatome von 12 bis 20, wobei die Menge an Hydroxyfettsäure im Bereich von 0,1 bis 20 Gewichts-% der Zusammensetzung und die Menge an Atractylenolid-I im Bereich von 0,01 bis 15 Gewichts-% der Zusammensetzung liegt.

## Revendications

1. Composition pour le soin de la personne comprenant :

a. de l'Atractylénolide-I ou une source d'Atractylénolide-I ; et
b. un acide gras hydroxy ayant un nombre d'atomes de carbone de 12 à 20, dans laquelle la quantité d'acide gras hydroxy se trouve dans l'intervalle de 0,1 à 20 % en masse de la composition, et la quantité d'Atractylénolide-I se trouve dans l'intervalle de 0,01 à 15 % en masse de la composition.

2. Composition selon la revendication 1, dans laquelle la quantité d'Atractylénolide-I se trouve dans l'intervalle de 0,02 à 10 % en masse, de préférence 0,1 à 5 % en masse de la composition.

3. Composition selon la revendication 1 ou 2, dans laquelle la source d'Atractylénolide-I est un extrait de plante.

4. Composition selon la revendication 3, dans laquelle la plante est *A tractylodes macrocephala.*

5. Composition selon l'une quelconque des revendications précédentes, dans laquelle la quantité d'acide gras hydroxy se trouve dans l'intervalle de 1 à 10 % en masse de la composition.

6. Composition selon l'une quelconque des revendications précédentes, dans laquelle l'acide gras hydroxy est l'acide hydroxystéarique.

7. Composition selon la revendication 6, dans laquelle l'acide hydroxystéarique est l'acide 12-hydroxystéarique (12HSA).

8. Composition selon l'une quelconque des revendications précédentes, dans laquelle la composition comprend de plus un agent bénéfique pour la peau supplémentaire.

9. Composition selon la revendication 8, dans laquelle l'agent bénéfique pour la peau est choisi parmi le niacinamide, picolinamide, isonicotinamide, hexyl résorcinol et des mélanges de ceux-ci.

10. Composition selon l'une quelconque des revendications précédentes, dans laquelle la composition comprend de 0,1 à 15 % en masse d'un écran solaire organique.

11. Composition selon l'une quelconque des revendications précédentes, dans laquelle la composition est une composition sans rinçage dans la forme d'une crème, lotion ou gel.

12. Composition pour le soin de la personne comprenant :

   a. un extrait *d'Atractylodes macrocephala* comprenant de l'Atractylénolide-I ; et
   b. un acide gras hydroxy ayant un nombre d'atomes de carbone de 12 à 20 dans laquelle la quantité d'acide gras hydroxy se trouve dans l'intervalle de 0,1 à 20 % en masse de la composition, et la quantité d'Atractylénolide-I se trouve dans l'intervalle de 0,01 à 15 % en masse de la composition.

13. Procédé non-thérapeutique pour la réduction d'hyperpigmentation sur la peau comprenant l'étape d'application d'une composition selon l'une quelconque des revendications 1 à 12.

14. Utilisation non-thérapeutique d'une composition selon l'une quelconque des revendications 1 à 12 pour réduire l'hyperpigmentation sur la peau.

15. Utilisation d'une composition comprenant :

   a. de l'Atractylénolide-I ou une source d'Atractylénolide-I ; et
   b. un acide gras hydroxy ayant un nombre d'atomes de carbone de 12 à 20, pour réduire l'hyperpigmentation sur la peau,

dans laquelle la quantité d'acide gras hydroxy se trouve dans l'intervalle de 0,1 à 20 % en masse de la composition, et la quantité d'Atractylénolide-I se trouve dans l'intervalle de 0,01 à 15 % en masse de la composition.

## REFERENCES CITED IN THE DESCRIPTION

**Patent documents cited in the description**

- CN 110420153
- CN 110664676
- CN 110559195
- WO 2014068470 A
- US 20090041875 A
- AU 2013202356 B
- KR 20160020253